# EUROPEAN PATENT SPECIFICATION

(11) **EP 4 054 507 B1**
(45) Date of publication and mention of the grant of the patent: **13.12.2023**
(21) Application number: 20885516.3
(22) Date of filing: 22.10.2020
(51) Int. Cl.: A61B 5/16, A61B 5/00, A61M 21/02, A61M 21/00

(54) **APPARATUS, SYSTEM AND METHOD FOR REDUCING STRESS**
VORRICHTUNG, SYSTEM UND VERFAHREN ZUR STRESSVERRINGERUNG
APPAREIL, SYSTÈME ET MÉTHODE POUR RÉDUIRE LE STRESS

(30) Priority: 05.11.2019 US 201962930639 P
(43) Date of publication of application: 14.09.2022
(73) Proprietor: Reflect Innovation Ltd., 4672562 Herzliya (IL)
(72) Inventor: SAPIR, Noga, Tel Aviv 6382156 (IL)
(74) Representative: AWA Denmark A/S
(86) International application number: PCT/IL2020/051105
(87) International publication number: WO 2021/090306

(56) References cited:
- CN-U- 204 910 071
- CN-U- 204 910 071
- JP-A- 2013 215 334
- US-A1- 2011 157 088
- US-A1- 2017 188 864
- US-B2- 9 081 489
- Sapir Noga: "REFLECT TEXTILE BIOFEEDBACK", , 23 September 2018 (2018-09-23), pages 1-4, XP055966131, Retrieved from the Internet: URL:https://www.nogasapir.com/reflect-text ile-biofeedback [retrieved on 2022-09-28]
- BioSign GmbH: "Qiu Manual", , 21 June 2017 (2017-06-21), pages 1-20, XP055966258, D-85570 Ottenhofen Retrieved from the Internet: URL:https://www.biosign.de/download_Qiu/GA W_Qiu_engl.pdf [retrieved on 2022-09-29]
- "Noga Sapir Reflect", vimeo, 30 August 2018 (2018-08-30), XP055822003, Retrieved from the Internet: URL:https://vimeo.com/287506958

## Description

### BACKGROUND

Medical research has shown that stress has an effect on health that can be harmful to the body. Stress, by definition, raises a person's blood pressure. Hypertension can increase the risk of a heart attack or stroke. Stress raises heart rate, blood pressure, blood glucose levels, and increased activity of the sympathetic nervous system and may result in a fight-or-flight response which is a physiological change in order to prepare the body for physical activity. Further, relaxed people can make better decisions and learn better.

There are many causes for stress, such as environmental factors such as living under overcrowded and increasingly noisy conditions, experiencing daily events such as coping with traffic, physical activity, undergoing major life changes such as a divorce or death of a loved one, stress in the work place and at home, and finally the use of chemical agents such as alcohol or drugs as a consequence of these stressors.

Stress response can be measured by measuring parameters, such as heart rate, blood pressure, skin conductivity and the like.

JP2013215334 discloses a autonomic nerve activity level display device; the device is spherically-shaped and comprises sensing electrodes configured to be in contact with a right hand and a left hand of a subject holding the device.

Sapir Noga: "REFLECT TEXTILE BIOFEEDBACK", 23 September 2018 (2018-09-23) discloses a biological feedback device shaped as a smart knit orb which is configured to respond to the changes in a user's stress level through changing intensities of light.

US2017188864 discloses a spherical handheld biosensor array device for physiological status monitoring which may use a light such as an LED to display the heart rate of a user by modulating the amplitude of the light emitted at the frequency of the user's heart rate.

BioSign GmbH: "Qiu Manual", 21 June 2017 (2017-06-21) discloses a spherically-shaped medical biofeedback device fitted with a sensitive pulse sensor and configured to be hold with one hand only; the device is further configured to measure the heart rhythm variability HRV and to provide an associated color-coded biofeedback signal in real-time.

However, the prior art still faces a number of limitations.

There is thus a need for an apparatus, a system and a method for reducing stress and/or inducing mindfulness.

### SUMMARY

The objects are at least achieved by an apparatus according to claim 1. Advantageous embodiments are described in the dependent claims.

The invention generally relates to a stress reducing and/or mindfulness inducing apparatus. The apparatus generally includes a substantially spherically shaped body "huggable" by two hands of a user, sensors for sensing the user's stress and/or mindfulness state, and an indicator for providing the user with an indication regarding his/her stress and/or mindfulness state.

Thus, there is provided a stress reducing and/or mindfulness inducing apparatus comprising: a substantially spherically shaped body "huggable" by two hands of a user; at least a first pair and a second pair of indentations located on an outer surface of the body, wherein each of the first and the second pair of indentations comprise two indentations, located on opposing sides of the body surface, and are suitable for placing corresponding fingers from each hand of the user, wherein each of the first and the second pair of indentations comprises one or more sensors, wherein the one or more sensors are configured to sense one or more parameters associated with the user's stress and/or mindfulness state, wherein the sensors are the same or different from each other; and an indicator having at least two modes of operation, a first mode being indicative of a first stress and/or mindfulness state of the user and a second mode being indicative of a second stress and/or mindfulness state of the user.

According to another aspect of some embodiments, there is provided an apparatus as described above, wherein the first pair of indentations comprises one indentation for an index finger of a left hand of the user and another indentation for the index finger of a right hand of the user, and wherein the second pair of indentations comprises one indentation for a middle finger of the left hand of the user and another indentation for the middle finger of the right hand of the user.

According to an aspect of some embodiments, there is provided an apparatus as described above, wherein the first pair of indentations comprises one indentation for any finger of a left hand of the user and another indentation for any finger of a right hand of the user, and wherein the second pair of indentations comprises one indentation for a finger of the left hand of the user and another indentation for the finger of the right hand of the user.

According to another aspect of some embodiments, there is provided an apparatus as described above, wherein the first pair of indentations comprises one indentation for an index, middle or ring finger of a left hand of the user and another indentation for the index, middle or ring finger of a right hand of the user, and wherein the second pair of indentations comprises one indentation for middle or ring finger of the left hand of the user and another indentation for the middle or ring finger of the right hand of the user.

According to another aspect of some embodiments, there is provided an apparatus as described above, wherein the apparatus further comprising a third pair of indentations, wherein the third pair of indentations comprises one indentation for a ring finger of the left hand of the user and another indentation for the ring finger of the right hand of the user.

In some embodiments there is provided a stress reducing and/or mindfulness inducing apparatus comprising: a substantially spherically shaped body "huggable" by two hands of a user; at least a first pair and a second pair of indentations located on an outer surface of the body, wherein each of the first and the second pair of indentations comprise two indentations, located on opposing sides of the body surface, and are suitable for placing corresponding fingers from each hand of the user, wherein the apparatus includes one or more sensors, wherein the one or more sensors are configured to sense one or more parameters associated with the user's stress and/or mindfulness state, wherein the sensors are the same or different from each other; and an indicator having at least two modes of operation, a first mode being indicative of a first stress and/or mindfulness state of the user and a second mode being indicative of a second stress and/or mindfulness state of the user.

According to another aspect of some embodiments, there is provided an apparatus as described above, wherein the apparatus further comprising a processor configured to receive a signal from the one or more sensors, to apply one or more algorithms to the data provided from the signal and to compute an output signal to the indicator.

According to another aspect of some embodiments, there is provided an apparatus as described above, wherein the apparatus is connectable to an external processor configured to receive a signal from the one or more sensors, to apply one or more algorithms to the data provided from the signal and to compute an output signal to the indicator.

According to another aspect of some embodiments, there is provided an apparatus as described above, wherein the one or more algorithms are artificial intelligence based.

According to another aspect of some embodiments, there is provided an apparatus as described above, wherein the apparatus further comprises an energy source.

According to another aspect of some embodiments, there is provided an apparatus as described above, wherein the apparatus further comprises an interface for external charging.

According to another aspect of some embodiments, there is provided an apparatus as described above, wherein the indicator is configured to provide visual, audio, tactile feedback information or any combination thereof.

According to another aspect of some embodiments, there is provided an apparatus as described above, wherein the apparatus, wherein the indicator is a light source. According to another aspect of some embodiments, there is provided an apparatus as described above, wherein the first mode of operation of the light source provide a first color output indicative of a first stress and/or first mindfulness state of the user and the second mode of operation of the light source provides a second color output, different from the first color, indicative of a second stress and/or a second mindfulness state of the user.

According to another aspect of some embodiments, there is provided an apparatus as described above, wherein the first mode of operation of the light source provides light at a first intensity, indicative of a first stress and/or first mindfulness state of the user and a second mode of operation of the light source provides light at a second intensity, different from the first intensity, indicative of a second stress and/or second mindfulness state of the user.

According to another aspect of some embodiments, there is provided an apparatus as described above, wherein the first mode of operation of the light source provides light pulses at a first frequency, indicative of a first stress and/or first mindfulness state of the user and the second mode of operation of the light source provides light pulses at a second frequency, different from the first frequency, indicative of a second stress, and/or second mindfulness state of the user.

According to another aspect of some embodiments, there is provided an apparatus as described above, wherein the indicator is embedded within the spherically shaped body.

According to another aspect of some embodiments, there is provided an apparatus as described above, wherein the indicator is covered by a transparent or semi-transparent cover.

According to another aspect of some embodiments, there is provided an apparatus as described above, wherein the indicator is configured to trigger vibration or expansion of essentially the entire body of the apparatus.

According to another aspect of some embodiments, there is provided an apparatus as described above, wherein the one or more sensors comprise GSR and ECG. According to another aspect of some embodiments, there is provided an apparatus as described above, wherein the one or more sensors comprises a GSR, ECG, PPG, accelerometer, thermometer, barometer, eye tracker, GPS, EMG, pupil size detector, pupil location and/or movement tracker, oximeter, pressure sensor, blood pressure sensor, gyroscope, microphone, camera, fingerprint sensor, humidity sensor, optical sensor, light sensor, photodiode, photoresistor, audiosensor, strain gauge or any combination thereof.

According to another aspect of some embodiments, there is provided an apparatus as described above, wherein the apparatus further comprises communication means functionally connectable to an external device.

According to another aspect of some embodiments, there is provided an apparatus as described above, wherein the communication means comprises wires, cellular communication, Bluetooth, IR, and/or Wi-Fi communication means.

According to another aspect of some embodiments, there is provided an apparatus as described above, wherein the outer surface of the apparatus comprises a soft shell.

According to another aspect of some embodiments, there is provided an apparatus as described above, wherein the soft shell comprises a soft fabric, a foam, a rubber, silicone, flexible plastic, cotton, wool or any combination thereof.

According to another aspect of some embodiments, there is provided an apparatus as described above, wherein the substantially spherically shaped body is in an oval shape or a ball shape.

According to another aspect of some embodiments, there is provided a system for a stress reducing and/or mindfulness inducing apparatus comprising: a substantially spherically shaped body "huggable" by two hands of a user, the substantially spherically shaped body comprises; at least a first pair and a second pair of indentations located on an outer surface of the body, wherein each of the first and the second pair of indentations comprise two indentations, located on opposing sides of the body surface, and are suitable for placing corresponding fingers from each hand of the user, wherein each of the first and the second pair of indentations comprises one or more sensors, wherein the one or more sensors are configured to sense one or more parameters associated with the user's stress and/or mindfulness state, wherein the sensors are the same or different from each other; and an indicator having at least two modes of operation, a first mode being indicative of a first stress and/or mindfulness state of the user and a second mode being indicative of a second stress and/or mindfulness state of the user; and a charger configured to provide power to the a spherically shaped body.

According to an aspect of the disclosure not forming part of the claimed invention, there is provided a biofeedback method of reducing stress and/or inducing mindfulness comprising: holding the apparatus as described above or the system of as described above using two hands and while placing the fingers in the designated indentations; receiving a first stress and/or mindfulness related indication from the indicator, self-adjusting a stress and/or mindfulness level; and receiving a second stress and/or mindfulness related indication from the indicator.

According to another aspect of some embodiments, there is provided a package that includes the apparatus or the system as described above and a leaflet with instructions regarding how to reach a reduced stress level and/or how to induce mindfulness.

Certain embodiments of the present disclosure may include some, all, or none of the above advantages. One or more other technical advantages may be readily apparent to those skilled in the art from the figures, descriptions, and claims included herein. Moreover, while specific advantages have been enumerated above, various embodiments may include all, some, or none of the enumerated advantages.

Unless otherwise defined, all technical and scientific terms used herein have the same meaning as commonly understood by one of ordinary skill in the art to which this disclosure pertains. In case of conflict, the patent specification, including definitions, governs. As used herein, the indefinite articles "a" and "an" mean "at least one" or "one or more" unless the context clearly dictates otherwise.

### BRIEF DESCIPTION OF THE DRAWINGS

The invention is defined by the appended claims and is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Figure 1 illustrates an isometric view of an exemplary apparatus for stress reducing and/or mindfulness inducing, according to some embodiments;
Figure 2 illustrates an isometric view of an exemplary system for stress reducing and/or mindfulness inducing, according to some embodiments;
Figure 3 depicts a user holding a stress reducing and/or mindfulness inducing apparatus with both his/her hands, according to some embodiments; and
Figure 4 depicts a block diagram of a stress reducing and/or mindfulness inducing system, according to some embodiments.

### DETAILED DESCRIPTION

There is provided herein a stress reducing and/or mindfulness inducing apparatus.

According to some embodiments, the term "inducing mindfulness" may include, for example, but not limited to, inducing meditation, guiding meditation, improving meditation state and/or assisting meditation, inducing focus, controlling/ influence/modulate alertness level (e.g., reducing alertness level, increasing alertness level or maintaining essentially constant alertness level), controlling/ influence/modulate excitation level, or any combination thereof. Each option presents a separate embodiment.

The apparatus has a substantially spherical shape and it is configured to be held ("hugged") by both hands of the user. The apparatus includes finger indentations. In the indentations, there are sensors (e.g., GSR, ECG or PPG) that measure one or more body parameters, such as heart rate, heart rate variability (HVR), blood pressure, skin conductivity, skin temperature and the like. The indentations are placed such that the hugging of the apparatus is comfortable, i.e. when the fingers are placed in the indentations, the palms of both hands of the user touch ("hug") the apparatus, which outer surface may comprise a soft shell. The apparatus includes an indicator, such as, but not limited to a display indicator, for example, a LED that produces changing light (changing color/frequency/intensity/pattern or any combination thereof) to provide feedback to the user based on the body's one or more parameter measured and thus assists the user to modulate his/her stress level or mindfulness state, for example, to calm down, to concentrate on a certain task, to control excitation level, to control alertness, to regulate breath, etc.

According to some embodiments, the invention provides a stress reducing and/or mindfulness inducing apparatus comprising: a substantially spherically shaped body "huggable" by two hands of a user; at least a first and a second pair of indentations located on an outer surface of the body, wherein each of the first and the second pair of indentations is suitable for placing corresponding fingers from each hand of the user, wherein each of the first and the second pair of indentations comprises one or more sensors, wherein the one or more sensors are configured to sense one or more parameters associated with the user's stress and/or mindfulness state, wherein the sensors are the same or different from each other; and an indicator having at least two modes of operation, the indicator is configured to provide the user with an indication regarding his/her stress and/or mindfulness state.

In some embodiments, the indicator may provide visual, audible, or tactile information or any combinations thereof. The indicator may be in a form of a light source, such as, in a form of text (wherein the parameter value is given as a number, out of a range of numbers, or as a word) or in some embodiments in a form of a light of different colors, frequencies, intensities, patterns, and the like, that represent the user's stress or mindfulness state. In some embodiments, the indicator is in an audible form and provides sounds in accordance with the user's stress or mindfulness state, such as a sentence, a comment, a word, music, a note, a certain tone, which may be, by a way of example: "you need to relax" or "you did well", "you are relaxed now", a certain music as indication of being focused, and the like.

In some embodiments, the indicator includes at least a light source or a sound source.

In some embodiments, the indicator is a lamp that lights up when activated in accordance with the stress or mindfulness state of the user. In some embodiments, the light source is a light emitting diode that lights up when activated in accordance with the stress or mindfulness state of the user.

In some embodiments, the indicator flashes a light when activated in accordance with the stress or mindfulness state.

In some embodiments, a first mode of operation of the indicator provides a first color output indicative of a first stress and/or first mindfulness state of the user, and a second mode of operation of the indicator provides a second color output, different from the first color, indicative of a second stress and/or a second mindfulness state of the user. For example, red may be used for high tension, blue for reduced tension, pink for being calm and green for being relaxed and mindful.

In some embodiments, a first mode of operation of the indicator provides light at a first intensity, indicative of a first stress and/or first mindfulness state of the user, and a second mode of operation of the indicator provides light at a second intensity, different from the first color, indicative of a second stress and/or second mindfulness state of the user.

In some embodiments, a first mode of operation of the indicator provides light pulses at a first frequency, indicative of a first stress and/or first mindfulness state of the user, and a second mode of operation of the indicator provides light pulses at a second frequency, different from the first color, indicative of a second stress and/or second mindfulness state of the user.

In some embodiments, the indicator may be embedded within the spherically shaped body. In some embodiments of the invention, the indicator is covered by a transparent or semi-transparent top.

For example, if the user is stressed, the indicator, which may be a light source, will send a red signal, and once the user relaxes, breathes slowly and/or thinks positive thoughts, the light turns to green. The indicator may have a scale of outputs such as different colors that represents stages of stress or mindfulness.

In some embodiments, the display indicator may be essentially the whole apparatus. In such case, the indication may include vibration or expansion/contraction of essentially the entire body of the apparatus.

In some embodiments, a processor is included in the apparatus or is connected to the apparatus by Wi-Fi or Bluetooth, for example. The processor is configured to receive a signal from the one or more sensors, to apply one or more algorithm to the data provided from the signal, and to compute an output signal to the indicator. In some embodiments the signal results from a combination of at least two parameters (for example, heart rate and skin conductance) and is computed as an output signal to the indicator.

In some embodiments, the apparatus disclosed herein may include an energy source. The energy source may be a battery, single use or rechargeable. In case where the apparatus includes rechargeable battery/batteries, the apparatus further includes an interface for external charging.

In some embodiments, the one or more sensors are capable of measuring skin conductance, respiration rate, heart rate, oxygen saturation, blood pressure, skin moisture and skin temperature. In some embodiments, the one or more sensors include GSR, PPG, ECG or any combination thereof. In some embodiments, the one or more sensors include GSR and ECG.

In some embodiments, the one or more sensors further comprises an accelerometer, thermometer, barometer, eye tracker, GPS, EMG, pupil size detector, pupil location/movement tracker, oximeter, pressure sensor, blood pressure sensor, gyroscope, microphone, camera, fingerprint sensor, humidity sensor, optical sensor, light sensor, photodiode, photoresistor, audiosensor, strain gauge, or any combination thereof.

The term "GSR" (galvanic skin response) refers, according to some embodiments, to a method of or a sensor for measuring the electrical conductance of the skin. Strong emotion can cause stimulus to the sympathetic nervous system that results in more sweat being secreted by the sweat glands. The GSR allows for spotting strong emotions by simply attaching two electrodes to two fingers.

The term "PPG" (photoplethysmography) refers, according to some embodiments, to sensor(s) that use a light-based technology to sense the rate of blood flow as controlled by the heart's pumping action.

The term "ECG sensors" refer, according to some embodiments, to sensors that are capable of delivering heart health metrics and are able to capture more comprehensive signals of overall heart performance, including both Heart Rate (HR) and Heart Rate Variability (HRV).

In some embodiments, the spherically shaped body is in the shape of a ball having a diameter between about 5cm-30cm, for example, between about 8cm-20cm. The apparatus may be provided in different sizes that may suit the size of the hand of a user.

In some embodiments, the outer surface of the apparatus comprises a soft shell.

In some embodiments, the apparatus is made of a soft fabric such as cotton, linen, wool, or from rubber, a cotton wool, or foam or silicone or any combination thereof.

In some embodiments, the indentations may have a length of between about 2cm-10cm and a depth of between about 0.5mm-20mm, for example, between about 1mm-15mm, 2mm-13mm and the like.

In some embodiments, each pair of indentations may have a different size.

In some embodiments, the size (e.g., length and depth) of the indentations may vary from an apparatus to an apparatus in accordance with the size of the apparatus so as to comply with the user's hands.

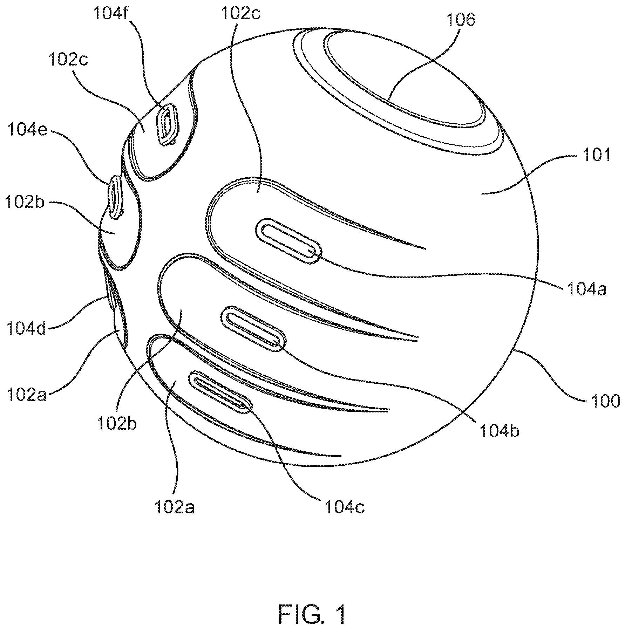

Reference is now made to **Figure 1****,** which schematically shows, in accordance with some embodiments, an isometric view of an exemplary apparatus **100** for stress reducing and/or mindfulness inducing. Apparatus **100** is shown herein as having ball shaped body **101,** though other shapes such as an oval shaped body are also included under the scope of this disclosure. Body **101** has a first **102a,** a second **102b** and a third **102c** pair of indentations located on an outer surface thereof. Each of first, second and third pair of indentations, **102a, 102b and 102c,** respectively, is suitable for placing corresponding index, middle and ring fingers of each hand of the user. Each of the first, second and third pair of indentations includes one or more sensors **104a, 104b, 104c, 104d, 104e, 104f,** wherein the one or more sensors **104a, 104b, 104c, 104d, 104e, 104f** are configured to sense one or more parameters associated with the user's stress and/or mindfulness state. The sensors may be one or more of GSR, PPG, ECG. By a way of example: sensors **104a** and **104b** are GSR sensors, sensors **104c** and **104e** are ECG sensors. As another example: sensor **104a** may include both GSR sensor and ECG sensor (not shown), such that sensor **104a** (GSR) and sensor **104b** are both GSR sensors configured to measure skin conductivity, and sensors **104a** (ECG), **104f, 104c** and **104e** (and optionally **104d)** are ECG sensors. Body **101** further includes an indicator **106** (e.g., a light source). Display indicator **106** is located on top of apparatus **100** and is functionally connected with one or more of sensors **104a, 104b, 104c, 104d, 104e, 104f.** Display indicator **106** has at least two modes of operation; indicator **106** is configured to provide the user with an indication regarding his/her stress and/or mindfulness state, e.g., "very stressed", "stress relieved", "relaxed", "excited", "focused", "distracted" etc. Apparatus **100** further includes a socket for a battery and optionally a charging interface (not shown).

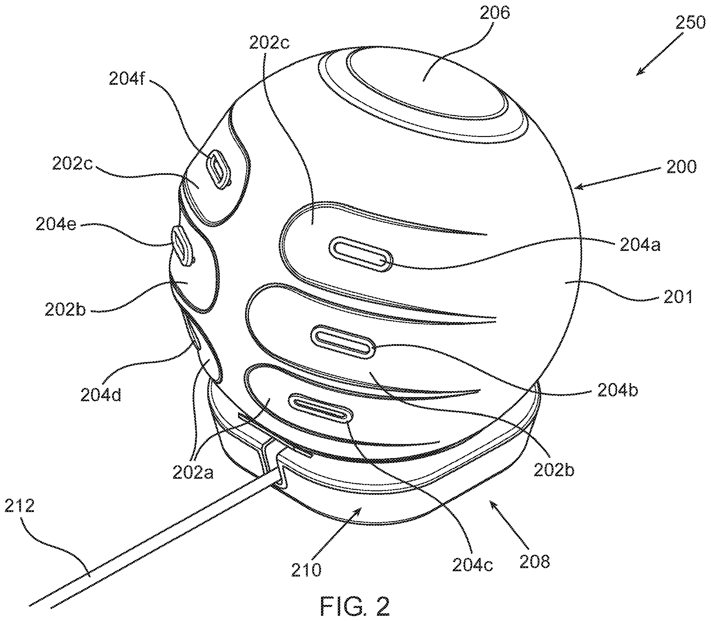

Reference is now made to **Figure 2****,** which schematically shows, in accordance with some embodiments, an isometric view of an exemplary system **250** including apparatus **200** and an energy source, e.g. a charger **208** having a cable **212.**

Apparatus **200** is shown herein as having ball shaped body **201,** though other shapes such as an oval shaped body are also included under the scope of this disclosure. Body **201** has a first **102a,** a second **202b** and a third **202c** pair of indentations located on an outer surface thereof. Each of first, second and third pair of indentations, **202a, 202b and 202c,** respectively, is suitable for placing corresponding index, middle and ring fingers of each hand of the user. Each of the first, second and third pair of indentations includes one or more sensors **204a, 204b, 204c, 204d, 204e, 204f,** wherein the one or more sensors **204a, 204b, 204c, 204d, 204e, 204f** are configured to sense one or more parameters associated with the user's stress and/or mindfulness state. The sensors may be one or more of GSR, PPG, ECG. By a way of example: sensors **204a** and **204b** are PPG, sensors **204c** and **204d** are GSR and sensors **204e** and **204f** are ECG. The sensors may be the same or different from each other. Body **201** further includes an indicator **206** (e.g., a display indicator, such as a light source). Indicator **206** is located on top of apparatus **200** and is functionally connected with one or more of sensors **204a, 204b, 204c, 204d, 204e, 204f.** Indicator **206** has at least two modes of operation; indicator **206** is configured to provide the user with an indication regarding his/her stress and/or mindfulness state e.g., "very stressed", "stress relieved", "relaxed", "excited", "focused", "distracted" etc. Apparatus **200** further includes an energy source, e.g. a charger **208,** having a base **210** suitable to the apparatus shape and a cable 212.

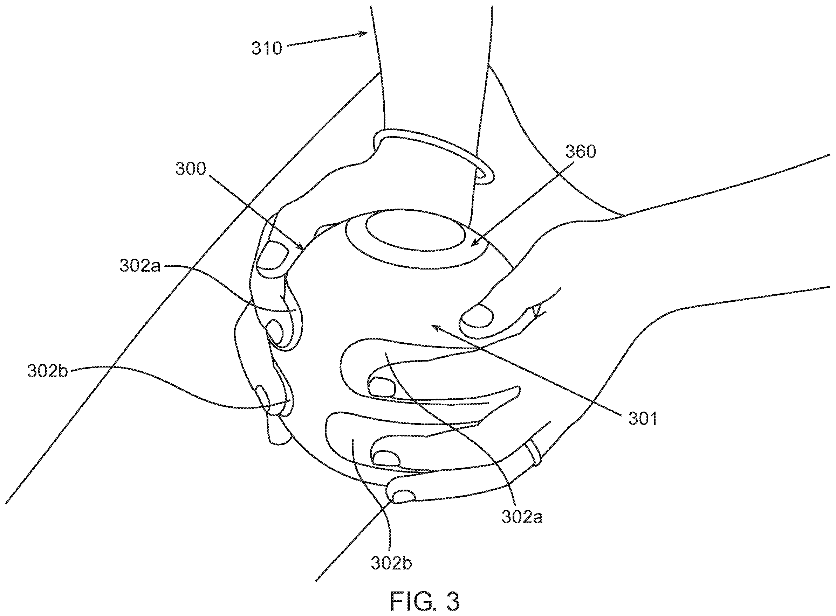

Reference is now made to **Figure 3****,** which depicts a user **310** holding a stress reducing and/or mindfulness inducing apparatus **300** with both his/her hands. Apparatus **300** is shown herein as having ball shaped body **301** though other shapes such as an oval shaped body are also included under the scope of this disclosure. Body **301** comprises a first **302a,** a second **302b** and a third (not shown) pair of indentations, respectively, and is suitable for placing corresponding index, middle and ring fingers of each hand of the user. Each of the first, second and third pair of indentations includes one or more sensors (not shown). The sensors may be one or more of GSR, PPG, ECG. The sensors may be the same or different from each other. Body **301** further includes a display indicator **360(light** source). Display indicator **360** is located on top of apparatus **300** and is functionally connected with one or more of sensors (not shown). Display indicator **360** has at least two modes of operation; display indicator **360** is configured to provide the user with an indication regarding his/her stress and/or mindfulness state e.g., "very stressed", "stress relieved", "relaxed", "excited", "focused", "distracted" etc. Apparatus **300** further includes an interface for an energy source, e.g., a charger or a battery (not shown).

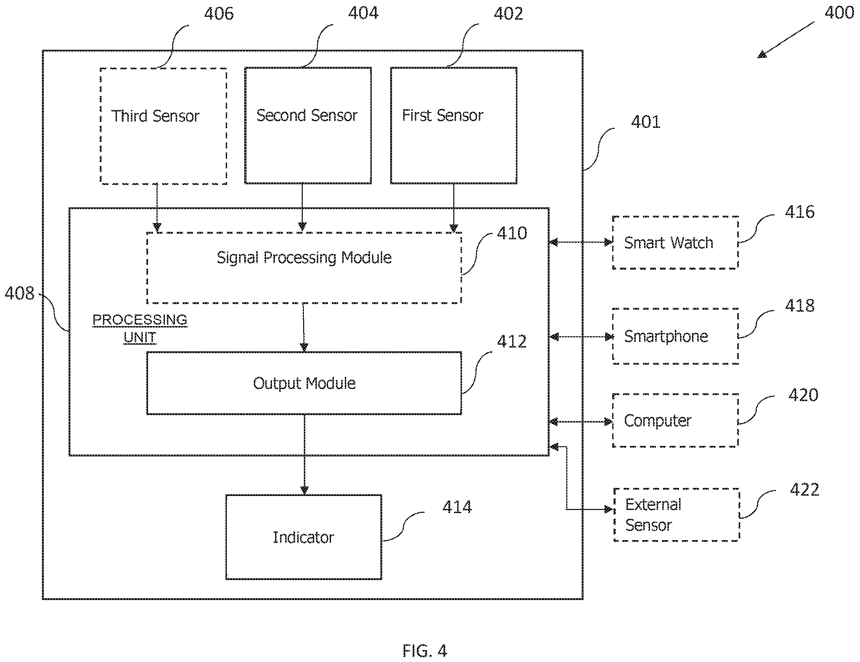

Reference is made now to **Figure 4****,** which depicts a block diagram of a stress reducing and/or mindfulness inducing system **400,** according to some embodiments. System **400** includes a handheld apparatus **401** (which may be similar to apparatus **100, 200** or **300** of **Figures 1****,** **2** and **3****,** respectively), which includes one or more sensors, such as sensors **402, 404** and **406** (and optionally more sensors, not shown), configured to sense one or more parameters associated with the user's stress and/or mindfulness state. Sensors **402, 404** and **406** may be similar, for example, to sensors **104a, 104b, 104c, 104d, 104e, 104f** of **Figure 1****.** System **400** further includes a processing unit **408** which includes a signal processing module **410** and output module **412.** Signal processing module **410** is configured to receive signals from the one or more sensors (such as sensors **402, 404** and/or **406),** apply one or more algorithms to the data computed from the signals and produce data relating to the user's stress and/or mindfulness state. Output module **412** is configured to provide an output signal indicative of the user's stress and/or mindfulness state, to an indicator **414.** According to some embodiments, signal processing module **410** may be external to handheld apparatus **401** (e.g., in and external device such as a mobile connectable device or a wearable device.

Apparatus **401** may communicate with other devices, such as, but not limited to, a smart watch **416,** a smartphone **418,** a computer **420,** and/or a remote server/cloud (not shown) by any type of communicationincluding, but not limited to, Wi-Fi, cellular communication, Bluetooth, IR, wired communication means, etc. Such devices may be utilized, for example, for controlling apparatus **401,** operating it, applying certain protocols to it, storing data, adding sensing capabilities, apply changes to algorithms, add display features, provide additional feedback, etc.

System **400** may further includes one or more external sensors, such as an external sensor **422,** in an external device, which may be for example, a wearable sensor or a mobile connectable device, a tablet or any computing module adding more sensing capabilities than provided by handheld apparatus **401.**

In some embodiments, there is provided a biofeedback method of reducing stress and or inducing mindfulness comprising the step of holding the apparatus as disclosed herein, in accordance with some embodiments, measuring a first stress and/or mindfulness state, relaxing and measuring a second stress and/or mindfulness state.

While certain features of the invention have been illustrated and described herein, many modifications, substitutions, and changes will now occur to those of ordinary skill in the art.

## Claims

1. A stress reducing and/or mindfulness inducing apparatus (100, 200, 300) comprising:
a substantially spherically shaped body (101, 201, 301) "huggable" by two hands of a user;
one or more sensors (104a-f, 204a-f) configured to measure one or more body parameters, wherein the one or more sensors (104a-f, 204a-f) are configured to sense one or more parameters associated with the user's stress and/or mindfulness state, wherein the sensors (104a-f, 204a-f) are the same or different from each other; and
an indicator (106, 206, 360) configured to provide feedback to the user based on the one or more body parameter measured and having at least two modes of operation, a first mode being indicative of a first stress and/or mindfulness state of the user and a second mode being indicative of a second stress and/or mindfulness state of the user, **characterized in that** the apparatus (100, 200, 300) further comprises at least a first pair (102a, 202a, 302a) and a second pair (102b, 202b, 302b) of indentations located on an outer surface of said body (101, 201, 301), wherein each of the first (102a, 202a, 302a) and the second (102b, 202b, 302b) pair of indentations comprise two indentations, located on opposing sides of said body surface, and are suitable for placing corresponding fingers from each hand of the user, wherein each of the first (102a, 202a, 302a) and the second (102b, 202b, 302b) pair of indentations comprises the one or more sensors (104a-f, 204a-f),
optionally wherein the first (102a, 202a, 302a) pair of indentations comprises one indentation for an index finger of a left hand of the user and another indentation for the index finger of a right hand of the user, and wherein the second pair of indentations (102b, 202b, 302b) comprises one indentation for a middle finger of the left hand of the user and another indentation for the middle finger of the right hand of the user.

2. The apparatus of claim 1, wherein the first pair of indentations (102a, 202a, 302a) comprises one indentation for an index finger of a left hand of the user and another indentation for the index finger of a right hand of the user, and wherein the second pair of indentations (102b, 202b, 302b) comprises one indentation for a middle finger of the left hand of the user and another indentation for the middle finger of the right hand of the user, further comprising a third pair of indentations (102c, 202c), wherein the third pair of indentations (102c, 202c) comprises one indentation for a ring finger of the left hand of the user and another indentation for the ring finger of the right hand of the user.

3. The apparatus of any one of claims 1-2, further comprising a processor configured to receive a signal from the one or more sensors (104a-f, 204a-f), to apply one or more algorithms to the data provided from the signal and to compute an output signal to the indicator (106, 206, 360) or wherein the apparatus is connectable to an external processor configured to receive a signal from the one or more sensors (104a-f, 204a-f), to apply one or more algorithms to the data provided from the signal and to compute an output signal to the indicator (106, 206, 360), optionally, wherein the one or more algorithms are artificial intelligence based.

4. The apparatus of any one of claims 1-3, wherein the apparatus further comprises an energy source (208) and/or wherein the apparatus further comprises an interface for external charging.

5. The apparatus of any one of claims 1-4, wherein the indicator (106, 206, 360) is configured to provide visual, audio, tactile feedback information or any combination thereof optionally, wherein the indicator is a light source (106, 206, 360).

6. The apparatus of claim 5, wherein the first mode of operation of the light source (106, 206, 360) provides a first color output indicative of a first stress and/or first mindfulness state of the user and the second mode of operation of the light source (106, 206, 360) provides a second color output, different from the first color, indicative of a second stress and/or a second mindfulness state of the user.

7. The apparatus of any one of claims 5 or 6, wherein the first mode of operation of the light source (106, 206, 360) provides light at a first intensity, indicative of a first stress and/or first mindfulness state of the user and a second mode of operation of the light source (106, 206, 360) provides light at a second intensity, different from the first intensity, indicative of a second stress and/or second mindfulness state of the user.

8. The apparatus of any one of claims 5-7, wherein the first mode of operation of the light source (106, 206, 360) provides light pulses at a first frequency, indicative of a first stress and/or first mindfulness state of the user and the second mode of operation of the light source (106, 206, 360) provides light pulses at a second frequency, different from the first frequency, indicative of a second stress, and/or second mindfulness state of the user.

9. The apparatus of any one of claims 1-8, wherein the indicator (106, 206, 360) is embedded within the spherically shaped body (101, 201, 301) and/or wherein the indicator (106, 206, 360) is covered by a transparent or semi-transparent cover.

10. The apparatus of any one of claims 1-2, wherein the indicator (106, 206, 360) is configured to trigger vibration or expansion of essentially the entire body of the apparatus.

11. The apparatus of any one of claims 1-10, wherein the one or more sensors (104a-f, 204a-f) comprises a GSR, ECG, PPG, accelerometer, thermometer, barometer, eye tracker, GPS, EMG, pupil size detector, pupil location and/or movement tracker, oximeter, pressure sensor, blood pressure sensor, gyroscope, microphone, camera, fingerprint sensor, humidity sensor, optical sensor, light sensor, photodiode, photoresistor, audiosensor, strain gauge or any combination thereof.

12. The apparatus of any one of claims 1-11, further comprising communication means functionally connectable to an external device, wherein the communication means comprises wires, cellular communication, Bluetooth, IR, and/or Wi-Fi communication means.

13. The apparatus of any one of claims 1-12, wherein the outer surface of the apparatus comprises a soft shell, optionally wherein the soft shell comprises a soft fabric, a foam, a rubber, silicone, flexible plastic, cotton, wool or any combination thereof.

14. The apparatus of any one of claims 1-13, wherein the substantially spherically shaped body (101, 201, 301) is in an oval shape or a ball shape.

15. A system for stress reducing and/or mindfulness inducing, comprising the apparatus of claim 1 and a charger (208) configured to provide power to the spherically shaped body (101, 201, 301).

## Patentansprüche

1. Einrichtung zur Stressverringerung und/oder zum Induzieren von Achtsamkeit (100, 200, 300), umfassend:
ein im Wesentlichen kugelförmiger Körper (101, 201, 301), der von einem Benutzer mit beiden Händen "umarmt" werden kann;
einen oder mehrere Sensoren (104a-f, 204a-f), die dafür ausgelegt sind, einen oder mehrere Körperparameter zu messen, wobei die ein oder mehreren Sensoren (104a-f, 204a-f) dafür ausgelegt sind, einen oder mehrere Parameter zu erfassen, die mit dem Benutzerzustand hinsichtlich Stress und/oder Achtsamkeit verknüpft sind, wobei die Sensoren (104a-f, 204a-f) identisch oder voneinander verschieden sind; und
einen Indikator (106, 206, 360), der dafür ausgelegt ist, eine Rückmeldung für den Benutzer bereitzustellen, basierend auf den gemessenen ein oder mehreren Körperparametern, und der über wenigstens zwei Betriebsmodi verfügt, wobei ein erster Modus einen ersten Stress- und/oder Achtsamkeitszustand des Benutzers anzeigt, und ein zweiter Modus einen zweiten Stress- und/oder Achtsamkeitszustand des Benutzers anzeigt, **dadurch gekennzeichnet, dass** die Einrichtung (100, 200, 300) ferner wenigstens ein erstes Paar (102a, 202a, 302a) und ein zweites Paar (102b, 202b, 302b) von Vertiefungen umfasst, die sich an einer Außenfläche des Körpers (101, 201, 301) befinden, wobei jedes von dem ersten (102a, 202a, 302a) und dem zweiten (102b, 202b, 302b) Paar von Vertiefungen zwei Vertiefungen umfasst, die sich an gegenüberliegenden Seiten der Körperoberfläche befinden und sich für das Platzieren entsprechender Finger von jeder Hand des Benutzers eignen, wobei jedes von dem ersten (102a, 202a, 302a) und dem zweiten (102b, 202b, 302b) Paar von Vertiefungen die ein oder mehreren Sensoren (104a-f, 204a-f) umfasst, wobei optional das erste (102a, 202a, 302a) Paar von Vertiefungen eine Vertiefung für einen Zeigefinger einer linken Hand des Benutzers und eine andere Vertiefung für den Zeigefinger einer rechten Hand des Benutzers umfasst, und wobei das zweite Paar von Vertiefungen (102b, 202b, 302b) eine Vertiefung für einen Mittelfinger der linken Hand des Benutzers und eine andere Vertiefung für den Mittelfinger der rechten Hand des Benutzers umfasst.

2. Einrichtung nach Anspruch 1, wobei das erste Paar von Vertiefungen (102a, 202a, 302a) eine Vertiefung für einen Zeigefinger einer linken Hand des Benutzers und eine andere Vertiefung für den Zeigefinger einer rechten Hand des Benutzers umfasst, und wobei das zweite Paar von Vertiefungen (102b, 202b, 302b) eine Vertiefung für einen Mittelfinger der linken Hand des Benutzers und eine andere Vertiefung für den Mittelfinger der rechten Hand des Benutzers umfasst, ferner umfassend ein drittes Paar von Vertiefungen (102c, 202c), wobei das dritte Paar von Vertiefungen (102c, 202c) eine Vertiefung für einen Ringfinger der linken Hand des Benutzers und eine andere Vertiefung für den Ringfinger der rechten Hand des Benutzers umfasst.

3. Einrichtung nach einem der Ansprüche 1-2, ferner umfassend einen Prozessor, der dafür ausgelegt ist, ein Signal von den ein oder mehreren Sensoren (104a-f, 204a-f) zu empfangen, um einen oder mehrere Algorithmen auf die Daten anzuwenden, die von dem Signal bereitgestellt werden, und um ein Ausgangssignal für den Indikator (106, 206, 360) zu berechnen, oder wobei die Einrichtung mit einem externen Prozessor verbunden werden kann, der dafür ausgelegt ist, ein Signal von den ein oder mehreren Sensoren (104a-f, 204a-f) zu empfangen, um einen oder mehrere Algorithmen auf die Daten anzuwenden, die von dem Signal bereitgestellt werden, um ein Ausgangssignal für den Indikator (106, 206, 360) zu berechnen, wobei die ein oder mehreren Algorithmen optional auf künstlicher Intelligenz basieren.

4. Einrichtung nach einem der Ansprüche 1-3, wobei die Einrichtung ferner eine Energiequelle (208) umfasst und/oder wobei die Einrichtung ferner eine Schnittstelle für externes Laden umfasst.

5. Einrichtung nach einem der Ansprüche 1-4, wobei der Indikator (106, 206, 360) dafür ausgelegt ist, visuelle, akustische, taktile Rückmeldeinformationen oder optional eine beliebige Kombination davon bereitzustellen, wobei der Indikator eines Lichtquelle (106, 206, 360) ist.

6. Einrichtung nach Anspruch 5, wobei der erste Betriebsmodus der Lichtquelle (106, 206, 360) eine erste Farbausgabe bereitstellt, die einen ersten Stress- und/oder einen ersten Achtsamkeitszustand des Benutzers anzeigt, und der zweite Betriebsmodus der Lichtquelle (106, 206, 360) eine zweite, von der ersten Farbe verschiedene Farbausgabe bereitstellt, die einen zweiten Stress- und/oder einen zweiten Achtsamkeitszustand des Benutzers anzeigt.

7. Einrichtung nach einem der Ansprüche 5 oder 6, wobei der erste Betriebsmodus der Lichtquelle (106, 206, 360) Licht mit einer ersten Intensität bereitstellt, was einen ersten Stress- und/oder einen ersten Achtsamkeitszustand des Benutzers anzeigt, und ein zweiter Betriebsmodus der Lichtquelle (106, 206, 360) Licht mit einer zweiten, von der ersten Intensität verschiedenen Intensität bereitstellt, was einen zweiten Stress- und/oder einen zweiten Achtsamkeitszustand des Benutzers anzeigt.

8. Einrichtung nach einem der Ansprüche 5-7, wobei der erste Betriebsmodus der Lichtquelle (106, 206, 360) Lichtimpulse mit einer ersten Frequenz bereitstellt, was einen ersten Stress- und/oder einen ersten Achtsamkeitszustand des Benutzers anzeigt, und der zweite Betriebsmodus der Lichtquelle (106, 206, 360) Lichtimpulse mit einer zweiten, von der ersten Frequenz verschiedenen Frequenz bereitstellt, was einen zweiten Stress- und/oder einen zweiten Achtsamkeitszustand des Benutzers anzeigt.

9. Einrichtung nach einem der Ansprüche 1-8, wobei der Indikator (106, 206, 360) in den kugelförmigen Körper (101, 201, 301) integriert ist und/oder wobei der Indikator (106, 206, 360) durch eine transparente oder halbtransparente Abdeckung abgedeckt ist.

10. Einrichtung nach einem der Ansprüche 1-2, wobei der Indikator (106, 206, 360) dafür ausgelegt ist, eine Vibration oder Ausdehnung des im Wesentlichen gesamten Körpers der Einrichtung auszulösen.

11. Einrichtung nach einem der Ansprüche 1-10, wobei die ein oder mehreren Sensoren (104a-f, 204a-f) umfassen: GSR, ECG, PPG, Beschleunigungsmesser, Thermometer, Barometer, Augenverfolger (Eye-Tracker), GPS, EMG, Pupillengrößendetektor, Pupillenpositions- und/oder Pupillenbewegungsverfolger, Oximeter, Drucksensor, Blutdrucksensor, Gyroskop, Mikrofon, Kamera, Fingerabdrucksensor, Feuchtigkeitssensor, Optosensor, Lichtsensor, Photodiode, Photowiderstand, Audio-Sensor, Dehnungsmesser oder eine beliebige Kombination davon.

12. Einrichtung nach einem der Ansprüche 1-11, ferner umfassend ein Kommunikationsmittel, das funktional mit einer externen Vorrichtung verbunden werden kann, wobei das Kommunikationsmittel drahtgebundene, auf Mobilkommunikation basierende, Bluetooth-, IR- und/oder WiFi-Kommunikationsmittel umfasst.

13. Einrichtung nach einem der Ansprüche 1-12, wobei die Außenfläche der Einrichtung eine weiche Hülle umfasst, wobei die weiche Hülle optional ein weiches Gewebe, Schaumstoff, Gummi, Silikon, flexiblen Kunststoff, Baumwolle, Wolle oder eine beliebige Kombination davon umfasst.

14. Einrichtung nach einem der Ansprüche 1-13, wobei der im Wesentlichen kugelförmige Körper (101, 201, 301) in einer ovalen Form oder eine Ballform vorliegt.

15. System zur Stressverringerung und/oder zum Induzieren von Achtsamkeit, umfassend die Einrichtung nach Anspruch 1 und ein Ladegerät (208), das dafür ausgelegt ist, den kugelförmigen Körper (101, 201, 301) mit Strom zu versorgen.

## Revendications

1. Appareil de réduction du stress et/ou d'induction de pleine conscience (100, 200, 300) comprenant :
un corps de forme sensiblement sphérique (101, 201, 301) « étreignable » par les deux mains d'un utilisateur ;
un ou plusieurs capteurs (104a-f, 204a-f) configurés pour mesurer un ou plusieurs paramètres corporels, le ou les capteurs (104a-f, 204a-f) étant configurés pour détecter un ou plusieurs paramètres associés à un état de stress et/ou de pleine conscience de l'utilisateur, les capteurs (104a-f, 204a-f) étant identiques ou différents les uns des autres ; et
un indicateur (106, 206, 360) configuré pour fournir un retour d'information à l'utilisateur sur la base du ou des paramètres corporels mesurés et ayant au moins deux modes de fonctionnement, un premier mode étant indicatif d'un premier état de stress et/ou de pleine conscience de l'utilisateur et un deuxième mode étant indicatif d'un deuxième état de stress et/ou de pleine conscience de l'utilisateur, **caractérisé en ce que** l'appareil (100, 200, 300) comprend en outre au moins une première paire (102a, 202a, 302a) et une deuxième paire (102b, 202b, 302b) d'indentations situées sur une surface extérieure dudit corps (101, 201, 301), chacune de la première (102a, 202a, 302a) et de la deuxième (102b, 202b, 302b) paire d'indentations comprenant deux indentations, situées sur les côtés opposés de ladite surface du corps, et étant appropriées pour placer les doigts correspondants de chaque main de l'utilisateur, chacune de la première (102a, 202a, 302a) et de la deuxième (102b, 202b, 302b) paire d'indentations comprenant le ou les capteurs (104a-f, 204a-f), éventuellement la première (102a, 202a, 302a) paire d'indentations comprenant une indentation pour l'index de la main gauche de l'utilisateur et une autre indentation pour l'index de la main droite de l'utilisateur, et la deuxième paire d'indentations (102b, 202b, 302b) comprenant une indentation pour le majeur de la main gauche de l'utilisateur et une autre indentation pour le majeur de la main droite de l'utilisateur.

2. Appareil selon la revendication 1, la première paire d'indentations (102a, 202a, 302a) comprenant une indentation pour l'index de la main gauche de l'utilisateur et une autre indentation pour l'index de la main droite de l'utilisateur, et la deuxième paire d'indentations (102b, 202b, 302b) comprenant une indentation pour le majeur de la main gauche de l'utilisateur et une autre indentation pour le majeur de la main droite de l'utilisateur, comprenant en outre une troisième paire d'indentations (102c, 202c), la troisième paire d'indentations (102c, 202c) comprenant une indentation pour l'annulaire de la main gauche de l'utilisateur et une autre indentation pour l'annulaire de la main droite de l'utilisateur.

3. Appareil selon l'une quelconque des revendications 1 et 2, comprenant en outre un processeur configuré pour recevoir un signal provenant du ou des capteurs (104a-f, 204a-f), pour appliquer un ou plusieurs algorithmes aux données fournies par le signal et pour calculer un signal de sortie vers l'indicateur (106, 206, 360) ou l'appareil pouvant être connecté à un processeur externe configuré pour recevoir un signal du ou des capteurs (104a-f, 204a-f), pour appliquer un ou plusieurs algorithmes aux données fournies par le signal et pour calculer un signal de sortie vers l'indicateur (106, 206, 360), éventuellement, le ou les algorithmes étant basés sur l'intelligence artificielle.

4. Appareil selon l'une quelconque des revendications 1 à 3, l'appareil comprenant en outre une source d'énergie (208) et/ou l'appareil comprenant en outre une interface pour la charge externe.

5. Appareil selon l'une quelconque des revendications 1 à 4, l'indicateur (106, 206, 360) étant configuré pour fournir des informations de rétroaction visuelles, audio, tactiles ou toute combinaison de celles-ci éventuellement, l'indicateur étant une source de lumière (106, 206, 360).

6. Appareil selon la revendication 5, le premier mode de fonctionnement de la source de lumière (106, 206, 360) fournissant une première sortie de couleur indicative d'un premier stress et/ou d'un premier état de pleine conscience de l'utilisateur et le deuxième mode de fonctionnement de la source de lumière (106, 206, 360) fournissant une deuxième sortie de couleur, différente de la première couleur, indicative d'un deuxième stress et/ou d'un deuxième état de pleine conscience de l'utilisateur.

7. Appareil selon l'une quelconque des revendications 5 ou 6, le premier mode de fonctionnement de la source de lumière (106, 206, 360) fournissant une lumière à une première intensité, indicative d'un premier stress et/ou d'un premier état de pleine conscience de l'utilisateur et un deuxième mode de fonctionnement de la source de lumière (106, 206, 360) fournissant une lumière à une deuxième intensité, différente de la première intensité, indicative d'un deuxième stress et/ou d'un deuxième état de pleine conscience de l'utilisateur.

8. Appareil selon l'une quelconque des revendications 5 à 7, le premier mode de fonctionnement de la source de lumière (106, 206, 360) fournissant des impulsions lumineuses à une première fréquence, indicative d'un premier stress et/ou d'un premier état de pleine conscience de l'utilisateur et le deuxième mode de fonctionnement de la source de lumière (106, 206, 360) fournissant des impulsions lumineuses à une deuxième fréquence, différente de la première fréquence, indicative d'un deuxième stress, et/ou d'un deuxième état de pleine conscience de l'utilisateur.

9. Appareil selon l'une quelconque des revendications 1 à 8, l'indicateur (106, 206, 360) étant intégré dans le corps de forme sphérique (101, 201, 301) et/ou l'indicateur (106, 206, 360) étant recouvert d'un couvercle transparent ou semi-transparent.

10. Appareil selon l'une quelconque des revendications 1 et 2, l'indicateur (106, 206, 360) étant configuré pour déclencher une vibration ou une expansion d'essentiellement la totalité du corps de l'appareil.

11. Appareil selon l'une quelconque des revendications 1 à 10, le ou les capteurs (104a-f, 204a-f) comprenant un GSR, un ECG, un PPG, un accéléromètre, un thermomètre, un baromètre, un traqueur oculaire, un GPS, un EMG, un détecteur de taille de pupille, un traqueur de localisation et/ou de mouvement de la pupille, un oxymètre, un capteur de pression, un capteur de pression artérielle, un gyroscope, un microphone, une caméra, un capteur d'indentations digitales, un capteur d'humidité, un capteur optique, un capteur de lumière, une photodiode, une photorésistance, un capteur audio, une jauge de contrainte ou toute combinaison de ceux-ci.

12. Appareil selon l'une quelconque des revendications 1 à 11, comprenant en outre des moyens de communication pouvant être connectés de manière fonctionnelle à un dispositif externe, les moyens de communication comprenant des fils, des moyens de communication cellulaire, Bluetooth, IR, et/ou Wi-Fi.

13. Appareil selon l'une quelconque des revendications 1 à 12, la surface extérieure de l'appareil comprenant une coque souple, éventuellement la coque souple comprenant du tissu souple, de la mousse, du caoutchouc, du silicone, du plastique souple, du coton, de la laine ou toute combinaison de ceux-ci.

14. Appareil selon l'une quelconque des revendications 1 à 13, le corps de forme sensiblement sphérique (101, 201, 301) ayant une forme ovale ou une forme de boule.

15. Système de réduction du stress et/ou d'induction de pleine conscience, comprenant l'appareil selon la revendication 1 et un chargeur (208) configuré pour fournir de l'énergie au corps de forme sphérique (101, 201, 301).
